# EUROPEAN PATENT SPECIFICATION

(11) **EP 2 616 038 B1**
(45) Date of publication and mention of the grant of the patent: **21.10.2015**
(21) Application number: 11754692.9
(22) Date of filing: 13.09.2011
(51) Int. Cl.: A61K 8/19, A61K 8/29, A61K 8/85, A61Q 1/02, A61Q 1/06, A61Q 17/04, C08J 3/22, C08K 3/22, C09C 1/36

(54) **COSMETIC COMPOSITION COMPRISING A DYESTUFF, SAID DYESTUFF AND COSMETIC TREATMENT PROCESS**
KOSMETISCHE ZUSAMMENSETZUNG MIT EINEM FARBSTOFF, BESAGTER FARBSTOFF UND KOSMETISCHES BEHANDLUNGSVERFAHREN
COMPOSITION COSMÉTIQUE COMPRENANT UNE SUBSTANCE COLORANTE, LADITE SUBSTANCE COLORANTE ET PROCÉDÉ COSMÉTIQUE DE TRAITEMENT

(30) Priority: 16.09.2010 US 383440 P; 14.09.2010 FR 1057317
(43) Date of publication of application: 24.07.2013
(73) Proprietor: L'Oréal, 75008 Paris (FR)
(72) Inventor: JEANNE-ROSE, Valérie, F-95100 Argenteuil (FR)
(74) Representative: Kromer, Christophe
(86) International application number: PCT/EP2011/065878
(87) International publication number: WO 2012/035029

(56) References cited:
- WO-A1-2008/038350
- WO-A1-2010/103215
- WO-A2-2010/042555
- WO-A2-2012/027728
- TW-A- 201 111 587
- US-A- 5 599 529
- US-A1- 2001 055 622
- US-A1- 2009 311 295
- US-A1- 2010 119 417

## Description

The present invention relates to cosmetic compositions comprising novel dyestuffs, to said dyestuffs and to a cosmetic treatment process employing them.

Makeup compositions generally contain dyestuffs, such as pigments or dyes, which give the applied makeup the desired colours. The number of dyestuffs that may be used in cosmetics is particularly limited, in particular because they must be registered on positive regulatory lists, which differ according to the economic zone (Europe, USA, Japan). Enlargement of the colorimetric range using permitted dyestuffs would thus be a major advantage, all the more so if they are dyestuffs of natural origin.

Moreover, mineral pigments are used in cosmetics mainly in antisun products or skin or hair makeup formulae. In the antisun formulae, use is generally made of submicron-sized pigments such as titanium oxide or zinc oxide. In makeup, use is made of white pigments such as titanium oxide or zinc oxide and coloured pigments such as quantum dots or metal particles, especially gold and/or silver particles.

It is known that certain dyestuffs from the prior art have a tendency, in certain cases, to run, which could, for example in the case of makeup compositions such as lipsticks or nail varnishes, result in a "streak" on the lips or the nails, and also to impairment of the quality of the makeup.

The objective of the present invention is to propose novel dyestuffs capable of being used in cosmetics that do not have the drawbacks of the prior art, especially that make it possible to obtain less migration into wrinkles or fine lines, hence less striations and for a therefore improved makeup effect to be obtained It has surprisingly been found that the encapsulation or coating of colouring compounds by/with biopolyesters made it possible to obtain dyestuffs having adjustable colorimetric properties.

Moreover, owing to the invention, it is possible to obtain dyestuffs in which the colouring compounds (soluble pigments or dyes) are perfectly immobilized, which means that the running and/or the "streak" are very particularly limited.

Thus, one subject of the present invention is a cosmetic process using a cosmetic composition comprising a dyestuff comprising a biopolyester and a colouring compound, said biopolyester comprising repeating units of formula (I), alone or as a mixture: 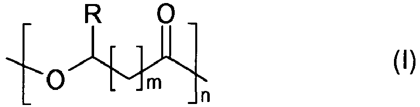 in which:
- R is a linear or branched saturated C1-C18 alkyl chain, or a hydrogen atom;
- m = 0 or 1; and
- n is between 450 and 1400;
said colouring compound being titanium oxide.

The dyestuffs according to the invention may have a very varied colour range in the visible and UV region, which may depend on the nature of the colouring compound, its concentration and/or the preparation process used, which makes it possible to use them in a particularly advantageous manner in cosmetic compositions, especially makeup compositions. Within the context of the present invention, white is considered to be a colour.

This is particularly noteworthy and advantageous given that these dyestuffs may have a virtually identical base composition, i.e. based on biopolyesters, which makes it possible to simplify their use in cosmetic compositions, said dyestuffs being readily interchangeable within the same base composition, thus making it possible to avoid adaptations of each composition to the nature of each dyestuff. The dyestuffs according to the invention also have the advantage of not running in common cosmetic media, whether they comprise common carbon-based oils or silicone oils.

Furthermore, dyestuffs have the advantage of giving good coverage.

The biopolyesters that may be used in the preparation of the dyestuffs according to the present invention are preferably polyesters of natural origin or derived from bioresources.

They comprise repeating units of formula (I), alone or as a mixture: 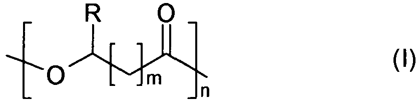 in which:
- R is a linear or branched saturated C1-C18 alkyl chain, or a hydrogen atom;
- m = 0 or 1; and
- n is between 450 and 1400.

Preferably, R is chosen from hydrogen, methyl and ethyl.

Preferably, m=1.

Preferably, R is a linear C1-C16 alkyl.

The biopolyesters according to the invention may preferably be homopolymers or copolymers comprising only monomers of formula (I).

The biopolyesters (or polymers) according to the invention preferably comprise, as unit of formula (I), the following repeating unit (II), alone or as a mixture: 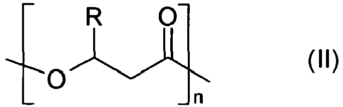 in which:
- R is a linear or branched saturated C1-C18 alkyl chain, or a hydrogen atom; and
- n is between 450 and 1400.

Preferably, R is chosen from hydrogen, methyl and ethyl.

The polymers according to the invention may be homopolymers (a single (II) unit) or copolymers comprising only monomers of formula (II). They may also be copolymers comprising, besides monomers of formula (II), additional monomers, which may preferably be of formula (I).

The polymers according to the invention may thus be advantageously chosen from homopolymers or copolymers of polyalkanoates; the polyalkanoates may especially comprise the following repeating units, alone or as a mixture:

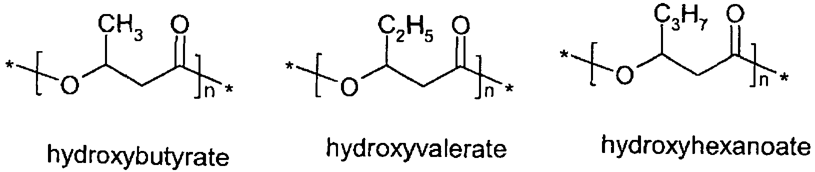

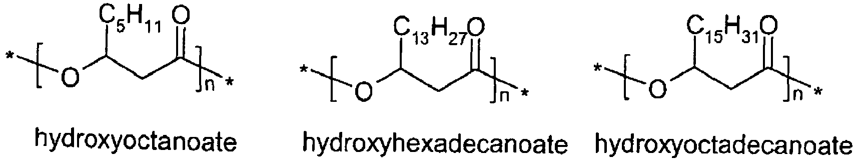

The polymers according to the invention may thus very particularly be chosen from homopolymers or copolymers of poly(hydroxybutyrate), and especially polyhydroxybutyrate or PHB, polyhydroxybutyrate-co-polyhydroxyvalerate or PHBV, polyhydroxybutyrate-co-polyhydroxyhexanoate or PHBHx, polyhydroxybutyrate-co-polyhydroxyoctanoate or PHBO, and polyhydroxybutyrate-co-polyhydroxyoctadecanoate or PHBOd.

The polymers according to the invention may also comprise, as unit of formula (I), a repeating unit of formula (III), alone or as a mixture: 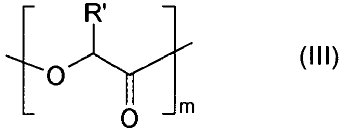 in which:
- R' is a linear or branched saturated C1-C18 alkyl chain, or a hydrogen atom; and
- m is between 450 and 1400.

Preferably, R' is chosen from hydrogen, methyl and ethyl, and better still R' = methyl (lactic acid).

Therefore, it may in particular be a question of polylactic acid homopolymers or polyalkanoate/polylactic acid copolymers, comprising at least the following repeating units:

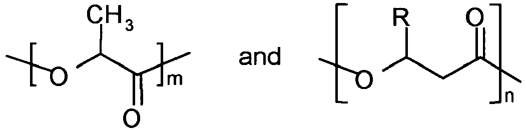

The polymers according to the invention may comprise, besides the units of formula (I), additional monomers, among which mention may be made of the ethylenically unsaturated monomers usually used in cosmetics, such as vinyl monomers, in particular (meth)acrylic monomers, especially (meth)acrylic acid and/or C1-C18 alkyl (meth)acrylate monomers.

Preferably, in the biopolyesters according to the invention, the monomers of formula (I) represent 30% to 100% by weight, especially 40% to 95% by weight, or even 55% to 90% by weight, and better still 70% to 80% by weight, of the total weight of the biopolyester, preferably 100% by weight.

The additional monomers may thus be absent (0%) or may be present in a proportion of from 0.1 % to 70% by weight, especially 5% to 60% by weight, or even 10% to 45% by weight, and better still 20% to 30% by weight.

Preferably, the biopolyesters according to the invention have a weight-average molecular weight (M_{w}) of between 40 000 and 100 000 g/mol (i.e. 40 to 100 kDa). Obviously, a mixture of biopolyesters according to the invention may be used.

Commercial products that may be mentioned include:
- polymers of the type (PHB, PHBV), especially Biopol from Monsanto, Metabolix from Metabolix, Enmat from Tianan, Biocycle from Copersucar, or Biomer L from Biomer,
- polymers of the type (PHBHx, PHBO, PHBOd), especially Nodax from Procter & Gamble;
- polylactic acid, especially Natureworks from Cargill-Dow, Lacty from Shimadzu, Lacea from Mitsui Chemicals, Heplon from Chronopol, CPLA from Dainippon Ink Chem; Eco Plastic from Toyota, PLA from Galactic, Treofan from Treofan, L-PLA from Purac, or Ecoloju from Mitsubishi.

The colouring compound is titanium oxide. Additional colouring compounds capable of being used for preparing the dyestuffs according to the invention are chosen from, alone or as a mixture:
- zinc oxide, silica,
- quantum dots,
- metal particles,
- plasmons or plasmonic particles.

The titanium oxide and the zinc oxide may be of nanometre or micron size.

Quantum dots are luminescent semiconductive nanoparticles capable of emitting, under light excitation, radiation with a wavelength of between 400 nm and 700 nm. These nanoparticles generally have a size of less than 10 nm.

They are known in the literature; in particular, they may be manufactured according to the processes described, for example, in patents US 6 225 198 or US 5 990 479, in the publications cited therein and also in the following publications: Dabboussi et al., "(CdSe)ZnS Core-Shell Quantum Dots: Synthesis and Characterisation of a Size Series of Highly Luminescent Nanocrystallites", Journal of Physical Chemistry B, vol. 101, 1997, pp. 9463-9475; and also in Peng, Xiaogang et al., "Epitaxial Growth of Highly Luminescent CdSe/CdS Core/Shell Nanocrystals with Photostability and Electronic Accessibility", Journal of the American Chemical Society, vol. 119, No. 30, pp. 7019-7029.

The quantum dots are preferably of core-shell structure and in particular of CdSe/CdS or CdSe/ZnS type.

The quantum dots are especially sold by the company Quantum Dots Corporation.

The metal particles may be of nanometre size; in particular they are gold and/or silver particles.

The expression "plasmons or plasmonic particles" is understood to mean a metal particle characterized by an electron density wave affecting the free electrons at the interface between a conductive material and a non-conductive material (for example a metal surface exposed to air).

This wave, similar to waves that travel across the surface of the metal, is produced, under certain conditions, by an incident light wave. A resonant interaction is obtained between the light and the plasmon.

Among the metals capable of constituting the plasmonic particles, mention may be made of: aluminium (Al), palladium (Pd), platinum (Pt), silver (Ag), copper (Cu), gold (Au) and zinc (Zn) and also mixtures and alloys thereof. Preferably, the metal of the plasmonic particles is chosen from the following metals: silver, aluminium, zinc and gold, and also alloys thereof. The alloys of several metals may be stoichiometric or non-stoichiometric. When several metals are used, it may be a continuous phase of metals such as an alloy, i.e. a continuous phase in which the metals are no longer dissociable, or a discontinuous phase of metals. The metallic surface may have a variety of shapes such as spherical, cylindrical, cubic, conical, ovoid, polyhedral, with four to ten faces, such as for example a perfect or truncated tetrahedron, a perfect or truncated cube, or a flake, especially an elliptical, triangular, pyramidal, or polyhedral flake. It may be smooth or rough.

The dyestuffs according to the invention are preferably in powder (solid) form or else in dispersion in an aqueous and/or organic solvent.

Preferably, the dyestuffs are in powder form, having a size of around 500 nm to 50 microns, and better still between 800 nm and 10 microns.

They preferably comprise 40% to 95% by weight of biopolyesters and 5% to 60% by weight of colouring compounds. Preferentially, they comprise 50% to 90% by weight, or even 55% to 85% by weight of biopolyesters and 10% to 50% by weight, or even 15% to 45% by weight of colouring compounds; they may also comprise additives, solvents or the like, for example which are already present in the colouring compounds.

Preferably, in the dyestuffs of the invention, the weight ratio between the colouring compounds and the biopolyesters is preferably between 0.05 and 1.2; especially between 0.1 and 1.0 and better still between 0.2 and 0.8; and preferentially between 0.45 and 0.8.

The dyestuffs according to the invention may be obtained via various processes. Mention may especially be made of an emulsification/solvent evaporation process, comprising the steps of:
- dissolution of the biopolyesters in a suitable solvent, especially dichloromethane and/or ethyl acetate;
- dissolution or dispersion, as may be the case, of the colouring compounds in a suitable solvent, especially water and/or dichloromethane;
- mixing and formation of a water-in-oil or oil-in-water emulsion in the presence of a suitable surfactant, especially sodium lauryl sulphate or polyglyceryl-4 diisostearate/polyhydroxystearate/sebacate;
- evaporation of the solvents.

The dyestuff may thus be obtained in the form of a dispersion (powder dispersed in a solvent) or in the form of a powder (dry powder, when all the solvents have been evaporated off).

It is also possible to prepare the dyestuffs according to the invention via a melting/milling process, comprising the steps of:
- heating the biopolyesters to a temperature above their melting point, so as to make them fluid in order to be able to mix them with the colouring compounds;
- adding the colouring compounds, as they are or in dispersion/solution in a suitable solvent;
- mixing, for example with a blender or an extruder;
- cooling to room temperature (20-25°C);
- milling, for example with a cryomill.

The dyestuff may thus be obtained in the form of a dispersion (powder dispersed in a solvent) or more preferentially in the form of a powder.

The dyestuffs according to the invention may advantageously be used in the cosmetics field.

They may be present in the cosmetic compositions in a proportion of from 0.1 % to 30% by weight, especially 0.5% to 25% by weight, or even 1% to 20% by weight, preferentially 3% to 15% by weight, and better still 5% to 10% by weight, relative to the total weight of the cosmetic composition.

Said cosmetic composition moreover comprises a cosmetically acceptable medium, i.e. a medium that is compatible with skin tissues such as facial or bodily skin, and keratin materials such as the hair, the eyelashes, the eyebrows and the nails.

The compositions may thus comprise, depending on the intended application, constituents that are common for this type of composition.

The composition according to the invention may advantageously comprise a liquid fatty phase, which may comprise at least one compound chosen from volatile or nonvolatile carbon-based, hydrocarbon-based, fluoro and/or silicone oils and/or solvents of mineral, animal, plant or synthetic origin, alone or as a mixture, provided that they form a stable, homogeneous mixture and are compatible with the intended use.

For the purposes of the invention, the term 'volatile' means any compound that is capable of evaporating on contact with keratin materials, or the lips, in less than one hour, at room temperature (25°C) and atmospheric pressure (1 atm). This volatile compound especially has a nonzero vapour pressure, at room temperature and atmospheric pressure, especially ranging from 0.13 Pa to 40 000 Pa (10⁻³ to 300 mmHg).

In contrast, the term 'nonvolatile' refers to a compound that remains on keratin materials or the lips at room temperature and atmospheric pressure for at least one hour and that especially has a vapour pressure of less than 10⁻³ mmHg (0.13 Pa).

Preferably, the cosmetically acceptable medium of the composition according to the invention may comprise, in a liquid fatty phase, at least one oil and/or solvent that may be chosen, alone or as a mixture, from:
1/ esters of monocarboxylic acids with monoalcohols and polyalcohols; advantageously, said ester is a C12-C15 alkyl benzoate or corresponds to the following formula: R'₁-COO-R'₂ in which:
   R'₁ represents a linear or branched alkyl radical of 1 to 40 carbon atoms and preferably of 7 to 19 carbon atoms, optionally comprising one or more ethylenic double bonds, optionally substituted, and the hydrocarbon-based chain of which may be interrupted with one or more heteroatoms chosen from N and O and/or one or more carbonyl functions, and
   R'₂ represents a linear or branched alkyl radical of 1 to 40 carbon atoms, preferably of 3 to 30 carbon atoms and better still of 3 to 20 carbon atoms, optionally comprising one or more ethylenic double bonds, optionally substituted, and the hydrocarbon-based chain of which may be interrupted with one or more heteroatoms chosen from N and O and/or one or more carbonyl functions.

   The term "optionally substituted" means that R'₁ and/or R'₂ may bear one or more substituents chosen, for example, from groups comprising one or more heteroatoms chosen from O and/or N, such as amino, amine, alkoxy or hydroxyl. Examples of groups R'₁ are those derived from fatty acids, preferably higher fatty acids chosen from the group formed from acetic acid, propionic acid, butyric acid, caproic acid, caprylic acid, pelargonic acid, capric acid, undecanoic acid, lauric acid, myristic acid, palmitic acid, stearic acid, isostearic acid, arachidic acid, behenic acid, oleic acid, linolenic acid, linoleic acid, oleostearic acid, arachidonic acid and erucic acid, and mixtures thereof.
   Preferably R'₁ is an unsubstituted branched alkyl group of 4 to 14 carbon atoms and preferably of 8 to 10 carbon atoms and R₂ is an unsubstituted branched alkyl group of 5 to 15 carbon atoms and preferably of 9 to 11 carbon atoms.
   Mention may be made in particular, preferably, of C₈-C₄₈ esters, optionally incorporating in their hydrocarbon-based chain one or more heteroatoms from among N and O and/or one or more carbonyl functions; and more particularly purcellin oil (cetostearyl octanoate), isononyl isononanoate, isopropyl myristate, isopropyl palmitate, 2-ethylhexyl palmitate, 2-octyldodecyl stearate, 2-octyldodecyl erucate, isostearyl isostearate, C₁₂ to C₁₅ alkyl benzoate, hexyl laurate, diisopropyl adipate; and heptanoates, octanoates, decanoates or ricinoleates of alcohols or of polyalcohols, for example of fatty alcohols, for instance propylene glycol dioctanoate, and also isopropyl N-lauroyl sarcosinate (especially Eldew-205SL from Ajinomoto); hydroxylated esters, for instance isostearyl lactate, diisostearyl malate; and pentaerythritol esters; branched C8-C16 esters, especially isohexyl neopentanoate.
2/ hydrocarbon-based plant oils with a high triglyceride content formed from fatty acid esters of glycerol, the fatty acids of which may have varied chain lengths from C₄ to C₂₄, these chains possibly being linear or branched, and saturated or unsaturated; these oils are especially wheat germ oil, corn oil, sunflower oil, shea oil, castor oil, sweet almond oil, macadamia oil, apricot oil, soybean oil, rapeseed oil, cottonseed oil, alfalfa oil, poppy oil, pumpkin oil, sesame oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passionflower oil, musk rose oil, jojoba oil, palm oil or beauty-leaf oil; or caprylic/capric acid triglycerides, such as those sold by the company Stearinerie Dubois or those sold under the names Miglyol 810^{®}, 812^{®} and 818^{®} by the company Dynamit Nobel.
3/ C6-C32 and especially C12-C26 alcohols, and especially monoalcohols, for instance oleyl alcohol, linoleyl alcohol, linolenyl alcohol, isostearyl alcohol, 2-hexyldecanol, 2-butyloctanol, 2-undecylpentadecanol and octyldodecanol;
4/ linear or branched, volatile or nonvolatile hydrocarbon-based oils, of synthetic or mineral origin, which may be chosen from hydrocarbon-based oils containing from 5 to 100 carbon atoms, and especially petroleum jelly, polydecenes, hydrogenated polyisobutenes such as Parleam, squalane and perhydrosqualene, and mixtures thereof.
   Mention may be made more particularly of linear, branched and/or cyclic C5-C48 alkanes, and preferentially branched C8-C16 alkanes, for instance C8-C16 isoalkanes of petroleum origin (also known as isoparaffins); especially decane, heptane, dodecane and cyclohexane; and also isododecane, isodecane and isohexadecane.
5/ volatile or nonvolatile silicone oils.

Volatile silicone oils that may be mentioned include volatile linear or cyclic silicone oils, especially those with a viscosity of less than 8 centistokes, and especially containing from 2 to 10 silicon atoms, these silicones optionally comprising alkyl or alkoxy groups containing from 1 to 22 carbon atoms; and in particular octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane and methylhexyldimethylsiloxane, and mixtures thereof.

The nonvolatile silicone oils that may be used according to the invention may be polydimethylsiloxanes (PDMS), polydimethylsiloxanes comprising alkyl or alkoxy groups, which are pendent and/or at the end of a silicone chain, these groups each containing from 2 to 24 carbon atoms, phenyl silicones, for instance phenyl trimethicones, phenyl dimethicones, phenyltrimethylsiloxydiphenylsiloxanes, diphenyl dimethicones, diphenylmethyldiphenyltrisiloxanes, and 2-phenylethyl trimethylsiloxysilicates.

The liquid fatty phase may also comprise additional oils and/or solvents, which may be chosen, alone or as a mixture, from:
- fluoro oils such as perfluoropolyethers, perfluoroalkanes such as perfluorodecalin, perfluoroadamantanes, perfluoroalkyl phosphate monoesters, diesters and triesters, and fluoro ester oils;
- oils of animal origin;
- C₆ to C₄₀ and especially C₁₀-C₄₀ ethers; propylene glycol ethers that are liquid at room temperature, such as propylene glycol monomethyl ether, propylene glycol monomethyl ether acetate and dipropylene glycol mono-n-butyl ether;
- C₈-C₃₂ fatty acids, for instance oleic acid, linoleic acid and linolenic acid, and mixtures thereof;
- difunctional oils, comprising two functions chosen from ester and/or amide and comprising from 6 to 30 carbon atoms, especially 8 to 28 carbon atoms and better still 10 to 24 carbon atoms, and 4 heteroatoms chosen from O and N; preferably, the amide and ester functions being in the chain;
- ketones that are liquid at room temperature (25°C) such as methyl ethyl ketone, methyl isobutyl ketone, diisobutyl ketone, isophorone, cyclohexanone and acetone;
- aldehydes that are liquid at room temperature, such as benzaldehyde and acetaldehyde.

The liquid fatty phase may represent 1% to 90% by weight of the composition, especially from 5% to 75% by weight, in particular from 10% to 60% by weight, or even from 25% to 55% by weight, of the total weight of the composition.

The composition according to the invention may also comprise at least one wax of plant, animal, mineral or synthetic origin, or even a silicone wax.

Mention may be made in particular, alone or as a mixture, of hydrocarbon-based waxes such as beeswax; carnauba wax, candelilla wax, ouricury wax, Japan wax, cork fibre wax or sugar cane wax; paraffin wax, lignite wax; microcrystalline waxes; lanolin wax; Montan wax; ozokerites; polyethylene waxes; waxes obtained by Fischer-Tropsch synthesis; hydrogenated oils, fatty esters and glycerides that are solid at 25°C. It is also possible to use silicone waxes, among which mention may be made of alkyl or alkoxy polymethylsiloxanes and/or polymethylsiloxane esters.

The composition according to the invention may also comprise one or more additional dyestuffs that may be chosen from pulverulent compounds, for instance pigments, fillers, nacres and glitter flakes, and/or fat-soluble or water-soluble dyes. The dyestuffs, especially pulverulent dyestuffs, may be present in the composition in a content of from 0.01 % to 50% by weight, preferably from 0.1 % to 40% by weight or even from 1 % to 30% by weight relative to the weight of the composition.

The term "pigments" should be understood as meaning white or coloured, mineral or organic particles of any shape, which are insoluble in the physiological medium, and which are intended to colour the composition.

The term "nacres" should be understood as meaning iridescent particles of any shape, especially produced by certain molluscs in their shell, or else synthesized. The pigments may be white or coloured, mineral and/or organic, and interference or non-interference pigments. Among the mineral pigments that may be mentioned are titanium dioxide, optionally surface-treated, zirconium oxides or cerium oxides, and also iron oxides or chromium oxides, manganese violet, ultramarine blue, chromium hydrate and ferric blue. Among the organic pigments that may be mentioned are carbon black, pigments of D & C type and lakes based on cochineal carmine or on barium, strontium, calcium or aluminium.

The nacreous pigments may be chosen from white nacreous pigments such as mica coated with titanium or with bismuth oxychloride, coloured nacreous pigments such as titanium mica with iron oxides, titanium mica especially with ferric blue or with chromium oxide, titanium mica with an organic pigment of the abovementioned type, and also nacreous pigments based on bismuth oxychloride. The fillers may be mineral or organic, and lamellar or spherical. Mention may be made of talc, mica, silica, kaolin, Nylon powder and polyethylene powder, poly-β-alanine powder and polyethylene powder, Teflon, lauroyllysine, starch, boron nitride, powders of tetrafluoroethylene polymers, hollow microspheres such as Expancel (Nobel Industrie), Polytrap (Dow Corning) and silicone resin microbeads (for example Tospearls from Toshiba), precipitated calcium carbonate, magnesium carbonate, magnesium hydrocarbonate, hydroxyapatite, hollow silica microspheres (Silica Beads from Maprecos), glass or ceramic microcapsules, and metal soaps derived from organic carboxylic acids containing from 8 to 22 carbon atoms and preferably from 12 to 18 carbon atoms, for example zinc, magnesium or lithium stearate, zinc laurate or magnesium myristate.

The fat-soluble dyes are, for example, Sudan red, DC Red 17, DC Green 6, β-carotene, soybean oil, Sudan brown, DC Yellow 11, DC Violet 2, DC Orange 5 and quinoline yellow. They may represent 0.01% to 20% and better still from 0.1 % to 6% of the weight of the composition.

The water-soluble dyes are, for example, beetroot juice or methylene blue, and may represent 0.01 % to 6% of the total weight of the composition.

The composition may also comprise other ingredients commonly used in cosmetic compositions, and which may be chosen from thickeners, gelling agents, antioxidants, fragrances, essential oils, preserving agents, cosmetic active agents, moisturizers, vitamins, ceramides, sunscreens, spreading agents, wetting agents, dispersants, antifoams, neutralizers, stabilizers, polymers and especially fat-soluble film-forming polymers; surfactants, emulsifiers; fillers, and mixtures thereof.

Needless to say, a person skilled in the art will take care to select this or these optional additional compound(s), and/or the amount thereof, such that the advantageous properties of the composition according to the invention are not, or are not substantially, adversely affected by the envisioned addition.

The compositions according to the invention may be in any form that is acceptable and common for a cosmetic composition, especially in the form of a suspension, a dispersion, especially of oil in water by means of vesicles; an optionally thickened or even gelled organic or oily solution; an oil-in-water, water-in-oil or multiple emulsion; a gel or a mousse; an oily or emulsified gel; a dispersion of vesicles, especially lipid vesicles; a two-phase or multiphase lotion; a spray; a lotion, a cream, a pomade, a soft paste, an ointment, a solid cast or moulded especially as a stick or in a dish, or a compacted solid.

A person skilled in the art may select the appropriate galenical form, and also the method for preparing it, on the basis of his general knowledge, taking into account the nature of the constituents used, especially their solubility in the support, and the intended application.

The compositions according to the invention may be used for caring for or making up keratin materials such as the hair, bodily or facial skin, the eyelashes, the eyebrows, the nails or the lips, and more particularly for making up the lips, the eyelashes, the hair and/or the face.

They may thus be in the form of a product for caring for or making up bodily or facial skin, the lips, the eyelashes, the eyebrows, the hair or the nails; an antisun or self-tanning product; a hair product, especially for dyeing, conditioning, cleansing shaping and/or caring for the hair; they are advantageously in the form of a makeup composition, especially lipstick, face powder, eyeshadow or foundation; or else an antisun composition.

A subject of the invention is also a cosmetic process for treating keratin materials, especially bodily or facial skin, the lips, the nails, the hair, the eyebrows and/or the eyelashes, comprising the application to said materials of a cosmetic composition as defined previously.

This process especially makes it possible to make up said keratin materials, in particular the lips, the face, the eyelids and/or the cheeks, by applying a lipstick, eyeshadow, face powder or foundation composition according to the invention; or else enables the antisun protection of said keratin materials, by applying an antisun composition according to the invention.

The invention is illustrated in greater detail in the following preparation examples.

### Example 1

A dyestuff according to the invention is prepared via a melting/milling process. 10 g of poly(3-hydroxybutyric acid-co-3-hydroxyvaleric acid) from Sigma-Aldrich (403121 containing 12% PHV) are heated to a temperature of 350°C in order to make them fluid. 10 g of titanium oxide (MT100AQ having a particle size of 4 nm) are added while hot, and the mixture is stirred by spatula and then left to cool to room temperature, down to 20-25°C, followed by cryomilling with dry ice or liquid nitrogen.

A white powder is obtained.

### Example 2

A dyestuff according to the invention is prepared via a melting/milling process.

Introduced into a 100 ml reactor are 7 g of titanium oxide (MT100T) and 8 g of 12% PHV poly(3-hydroxybutyric acid-co-3-hydroxyvaleric acid). The temperature of the heating mantle is set at 200°C; the mixture is stirred by spatula until it solidifies. It is left to cool before cryomilling.

A white powder is obtained.

### Example 3 (not according to the invention)

A dyestuff according to the invention is prepared via a melting/milling process.

Introduced into a 100 ml reactor are 1.2 g of gold plasmonic particles of red colour, and 8 g of 12% PHV poly(3-hydroxybutyric acid-co-3-hydroxyvaleric acid). The temperature of the heating mantle is set at 200°C; the mixture is stirred by spatula until it solidifies. It is left to cool before cryomilling. A red powder is obtained.

### Example 4

A cosmetic composition of lipstick type is prepared, comprising (% by weight):
- 5% dyestuff of Example 3
- 15% polyethylene wax
- 5% octyldodecanol
- 75% Parleam

### Example 5

A cosmetic composition of lipstick type is prepared, comprising (% by weight):
- 1% dyestuff of Example 2
- 4% lithol red B
- 15% polyethylene wax
- 5% octyldodecanol
- 75% Parleam

### Example 6

A cosmetic eyeshadow composition is prepared, comprising (% by weight):
- Dyestuff of Example 3 10%
- Duochrome BG 20%
- Magnesium stearate 2%
- Phenyl trimethicone/triisostearine (50/50) 6%
- Talc qs 100%

### Example 7

A cosmetic hair styling gel composition is prepared, comprising (% by weight):
- Dyestuff of Example 1 1%
- Hydroxypropyl guar (Jaguar HP 105 from Rhodia) 4%
- Water qs 100%

### Example 8

A foundation is prepared, comprising (in % by weight):
- Magnesium sulphate 1.5
- Sodium carboxymethyl cellulose 0.5
- Modified hectorite 1
- Glycerol 5
- Mixture of oxyethylenated polymethylcetyldimethylmethylsiloxane, polyglyceryl isostearate (4 moles) and hexyl laurate 9
- Mixture of ethylene glycol acetyl stearate and glyceryl tristearate 0.3
- 1,2-pentanediol 3
- Iron oxides 2.3
- Titanium oxide 18
- Dyestuff of Example 3 4.5
- Polydimethylsiloxane (viscosity: 5 cst) 6
- Cyclopentadimethylsiloxane 16
- Water qs 100%

### Example 9

An antisun composition is prepared, comprising (% by weight):

| *Phase A* | |
|---|---|
| Polydimethylsiloxane | 0.5 |
| Preservatives | qs |
| Stearic acid | 1.5 |
| Glyceryl monostearate/PEG stearate (100 EO) mixture | 1 |
| Cetylstearyl glucoside/cetylstearyl alcohol mixture | 2 |
| C12/C15 alkyl benzoate | 5 |
| Isohexadecane | 1 |
| Cetyl alcohol | 0.5 |
| Butyl Methoxydibenzoylmethane | 2 |
| Octocrylene | 9 |
| Drometrizole Trisiloxane | 1 |
| Bis-Ethylhexyloxyphenol Methoxyphenyl Triazine | 1 |
| Dyestuff of Example 1 | 3 |

| *Phase B* | |
|---|---|
| Deionized water | qs 100% |
| Sequestrant | 0.1 |
| Glycerol | 5 |
| Xanthan gum | 0.2 |
| Monocetyl phosphate | 1 |

| *Phase C* | |
|---|---|
| Acrylic acid/stearyl methacrylate copolymer | 0.2 |
| Triethanolamine | qs |

## Claims

1. Cosmetic process for treating keratin materials comprising the application to said materials of a cosmetic composition comprising, in a cosmetically acceptable medium, at least one dyestuff comprising a biopolyester and a colouring compound, said biopolyester comprising repeating units of formula (I), alone or as a mixture: 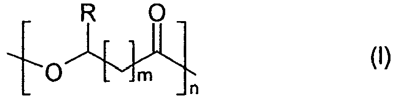 in which:
- R is a linear or branched saturated C1-C18 alkyl chain, or a hydrogen atom;
- m = 0 or 1; and
- n is between 450 and 1400;
said colouring compound being titanium oxide and being encapsulated or coated with the said biopolyester.

2. Cosmetic process according to Claim 1, in which in the dyestuff the weight ratio between said colouring compound and said biopolyester is between 0.05 and 1.2; especially between 0.1 and 1.0 and better still between 0.2 and 0.8; and preferentially between 0.45 and 0.8.

3. Cosmetic process according to one of the preceding claims, in which in the dyestuff the biopolyester comprises, as unit of formula (I), a repeating unit of formula (II), alone or as a mixture: 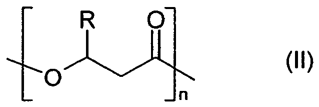 in which:
- R is a linear or branched saturated C1-C18 alkyl chain, or a hydrogen atom; and
- n is between 450 and 1400.

4. Cosmetic process according to one of the preceding claims, in which in the dyestuff the biopolyester is chosen from homopolymers or copolymers of polyalkanoates which may comprise the following repeating units, alone or as a mixture:

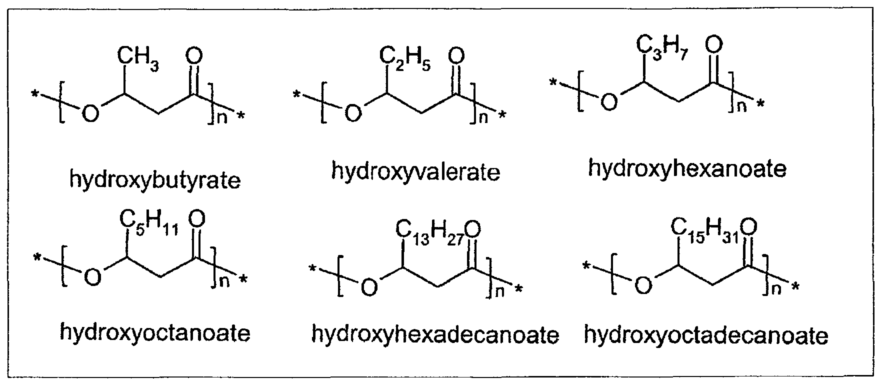

5. Cosmetic process according to one of the preceding claims, in which in the dyestuff the biopolyester comprises, as unit of formula (I), a repeating unit of formula (III), alone or as a mixture: 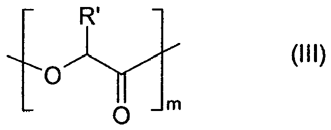 in which:
- R' is a linear or branched saturated C1-C18 alkyl chain, or a hydrogen atom; and
- m is between 450 and 1400.

6. Cosmetic process according to one of the preceding claims, in which in the dyestuff the monomers of formula (I) represent 30% to 100% by weight, especially 40% to 95% by weight, or even 55% to 90% by weight, and better still 70% to 80% by weight, of the total weight of the biopolyester, preferably 100% by weight.

7. Cosmetic process according to one of the preceding claims, in which the dyestuff is in powder (solid) form, having a size of around 500 nm to 50 microns, and better still between 800 nm and 10 microns.

8. Cosmetic process according to one of the preceding claims, in which the dyestuff comprises 40% to 95% by weight of biopolyesters and 5% to 60% by weight of colouring compounds.

9. Cosmetic process according to one of the preceding claims, in which the dyestuff is present in the composition in a proportion of from 0.1 % to 30% by weight, especially 0.5% to 25% by weight, or even 1% to 20%, preferentially 3% to 15% by weight, and better still 5% to 10% by weight, relative to the total weight of the cosmetic composition.

10. Cosmetic process according to one of the preceding claims, in which the cosmetically acceptable medium comprises at least one ingredient chosen from volatile or nonvolatile, carbon-based, hydrocarbon-based, fluoro and/or silicone oils and/or solvents of mineral, animal, plant or synthetic origin; waxes of plant, animal, mineral or synthetic origin, or silicone waxes; pigments, fillers, nacres and glitter flakes, fat-soluble or water-soluble dyes; thickeners, gelling agents, antioxidants, fragrances, essential oils, preserving agents, cosmetic active agents, moisturizers, vitamins, ceramides, sunscreens, spreading agents, wetting agents, dispersants, antifoams, neutralizers, stabilizers, polymers and especially fat-soluble film-forming polymers; surfactants, emulsifiers; fillers, and mixtures thereof.

11. Cosmetic process according to one of the preceding claims, in which the composition is in the form of a product for caring for or making up bodily or facial skin, the lips, the eyelashes, the eyebrows, the hair or the nails, an antisun or self-tanning product, or a haircare product, especially for dyeing, conditioning, cleansing, shaping and/or caring for the hair.

12. Cosmetic process according to one of the preceding claims, in which the composition is in the form of lipstick, face powder, eyeshadow or foundation; or an antisun composition.

13. Cosmetic process according to one of the preceding claims, for caring for or making up keratin materials, especially bodily or facial skin, the lips, the nails, the hair, the eyebrows and/or the eyelashes.

## Patentansprüche

1. Kosmetikverfahren zur Behandlung von Keratinsubstanzen, bei dem man eine Kosmetikzusammensetzung, die mindestens einen Farbstoff, der einen Biopolyester und eine färbende Verbindung umfasst, in einem kosmetisch unbedenklichen Medium umfasst, auf die Keratinsubstanzen aufbringt, wobei der Biopolyester Wiederholungseinheiten der Formel (I), allein oder als Mischung, 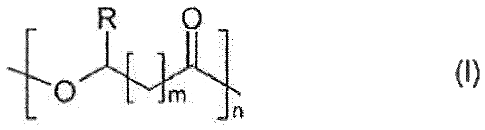 umfasst, worin
- R eine gerade oder verzweigte gesättigte C1-C18-Alkylkette oder ein Wasserstoffatom bedeutet;
- m = 0 oder 1; und
- n zwischen 450 und 1400 ist;
wobei die färbende Verbindung Titanoxid ist und mit dem Biopolyester verkapselt oder beschichtet ist.

2. Kosmetikverfahren nach Anspruch 1, in dem das Gewichtsverhältnis zwischen der färbenden Verbindung und dem Biopolyester in dem Farbstoff zwischen 0,05 und 1,2, speziell zwischen 0,1 und 1,0 und noch besser zwischen 0,2 und 0,8 und vorzugsweise zwischen 0,45 und 0,8 beträgt.

3. Kosmetikverfahren nach einem der vorhergehenden Ansprüche, in dem der Biopolyester in dem Farbstoff als Einheit der Formel (I) eine Wiederholungseinheit der Formel (II), 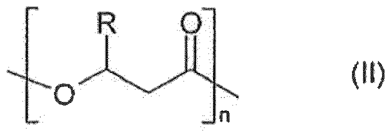 allein oder als Mischung, umfasst, worin
- R eine gerade oder verzweigte gesättigte C1-C18-Alkylkette oder ein Wasserstoffatom bedeutet;
- n zwischen 450 und 1400 ist.

4. Kosmetikverfahren nach einem der vorhergehenden Ansprüche, in dem der Biopolyester in dem Farbstoff aus Homopolymeren oder Copolymeren von Polyalkanoaten, die die folgenden Wiederholungseinheiten, allein oder als Mischung, umfassen können:

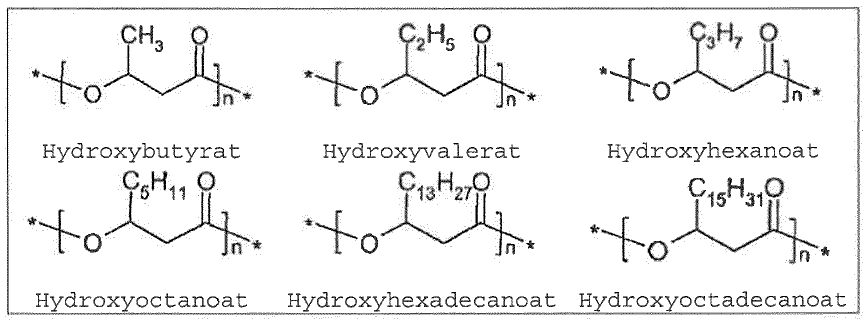

5. Kosmetikverfahren nach einem der vorhergehenden Ansprüche, in dem der Biopolyester in dem Farbstoff als Einheit der Formel (I) eine Wiederholungseinheit der Formel (III), 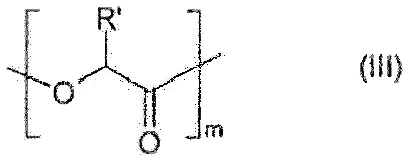 allein oder als Mischung, umfasst, worin
- R' eine gerade oder verzweigte gesättigte C1-C18-Alkylkette oder ein Wasserstoffatom bedeutet; und
- m zwischen 450 und 1400 ist.

6. Kosmetikverfahren nach einem der vorhergehenden Ansprüche, in dem die Monomere der Formel (I) in dem Farbstoff 30 Gew.-% bis 100 Gew.-%, speziell 40 Gew.-% bis 95 Gew.-%, ja sogar 55 Gew.-% bis 90 Gew.-%, noch besser 70 Gew.-% bis 80 Gew.-%, vorzugsweise 100 Gew.-%, des Gesamtgewichts des Biopolyesters ausmachen.

7. Kosmetikverfahren nach einem der vorhergehenden Ansprüche, in dem der Farbstoff in Pulverform (fester Form) mit einer Größe von ungefähr 500 nm bis 50 Mikrometern, noch besser zwischen 800 nm und 10 Mikrometern, vorliegt.

8. Kosmetikverfahren nach einem der vorhergehenden Ansprüche, in dem der Farbstoff 40 Gew.-% bis 95 Gew.-% Biopolyester und 5 Gew.-% bis 60 Gew.-% färbende Verbindungen umfasst.

9. Kosmetikverfahren nach einem der vorhergehenden Ansprüche, in dem der Farbstoff in der Zusammensetzung in einem Verhältnis von 0,1 Gew.-% bis 30 Gew.-%, speziell 0,5 Gew.-% bis 25 Gew.-%, ja sogar 1 Gew.-% bis 20 Gew.-%, vorzugsweise 3 Gew.-% bis 15 Gew.-%, noch besser 5 Gew.-% bis 10 Gew.-%, in Bezug auf das Gesamtgewicht der Kosmetikzusammensetzung ausmacht.

10. Kosmetikverfahren nach einem der vorhergehenden Ansprüche, in dem das kosmetisch unbedenkliche Medium mindestens einen Bestandteil, ausgewählt aus der folgenden Gruppe, umfasst: flüchtige oder nichtflüchtige, kohlenstoffbasierte, kohlenwasserstoffbasierte Fluor- und/oder Silikonöle und/oder Lösungsmittel mineralischen, tierischen, pflanzlichen oder synthetischen Ursprungs; Wachse pflanzlichen, tierischen, mineralischen oder synthetischen Ursprungs, oder Silikonwachse; Pigmente, Füllstoffe, Perlglanzstoffe und Glitzerflocken, fettlösliche oder wasserlösliche Farbmittel; Verdickungsmittel, Geliermittel, Antioxidantien, Duftstoffe, ätherische Öle, Konservierungsstoffe, kosmetische Wirkstoffe, feuchtigkeitsspendende Stoffe, Vitamine, Ceramide, Sonnenschutzfilter, Spreitmittel, Netzmittel, Dispergiermittel, Schaumhemmer, Neutralisierungsmittel, Stabilisierungsmittel, Polymere und insbesondere fettlösliche filmbildende Polymere; Tenside; Emulgatoren; Füllstoffe, und Mischungen davon.

11. Kosmetikverfahren nach einem der vorhergehenden Ansprüche, in dem die Zusammensetzung in Form eines Produkts für die Pflege oder das Schminken von Körper- oder Gesichtshaut, Lippen, Wimpern, Augenbrauen, des Haars oder der Nägel, als Sonnenschutzmittel oder Selbstbräunungsmittel oder als Haarpflegemittel, insbesondere für das Färben, Konditionieren, Reinigen, Formen und/oder Pflegen des Haars vorliegt.

12. Kosmetikverfahren nach einem der vorhergehenden Ansprüche, in dem die Zusammensetzung in Form eines Lippenstifts, eines Gesichtspuders, eines Lidschattens oder einer Make-Up-Grundlage oder als Sonnenschutzzusammensetzung vorliegt.

13. Kosmetikverfahren nach einem der vorhergehenden Ansprüche für die Pflege oder das Schminken von Keratinsubstanzen, insbesondere Körper- oder Gesichtshaut, der Lippen, der Nägel, des Haars, der Augenbrauen und/oder der Wimpern.

## Revendications

1. Procédé cosmétique destiné à traiter des matières kératiniques, comprenant l'application auxdites matières d'une composition cosmétique comprenant, dans un milieu cosmétiquement acceptable, au moins une substance colorante comprenant un bio-polyester et un composé colorant, ledit bio-polyester comprenant des motifs répétitifs de formule (I), seuls ou en mélange : 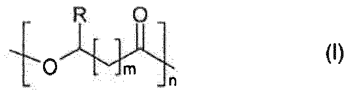 où
- R est une chaîne C1-C18 alkyle linéaire ou ramifiée, ou un atome d'hydrogène ;
- m= 0 ou 1 ; et
- n est compris entre 450 et 1400 ;
ledit composé colorant étant de l'oxyde de titane et étant encapsulé ou enrobé par ledit bio-polyester.

2. Procédé cosmétique selon la revendication 1, dans lequel, dans la substance colorante, le rapport pondéral entre ledit composé colorant et ledit bio-polyester est compris entre 0,05 et 1,2 ; notamment entre 0,1 et 1,0 et mieux encore entre 0,2 et 0,8 ; et préférablement entre 0,45 et 0,8.

3. Procédé cosmétique selon l'une des revendications précédentes, dans lequel, dans la substance colorante, le bio-polyester comprend, comme motif de formule (I), un motif répétitif de formule (II), seul ou en mélange : 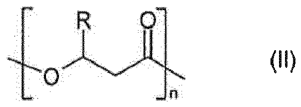 où
- R est une chaîne C1-C18 alkyle linéaire ou ramifiée, ou un atome d'hydrogène ; et
- n est compris entre 450 et 1400.

4. Procédé cosmétique selon l'une des revendications précédentes, dans lequel, dans la substance colorante, le bio-polyester est choisi parmi des homopolymères ou des copolymères de polyalcanoates pouvant comprendre les motifs répétitifs suivants, seuls ou en mélange

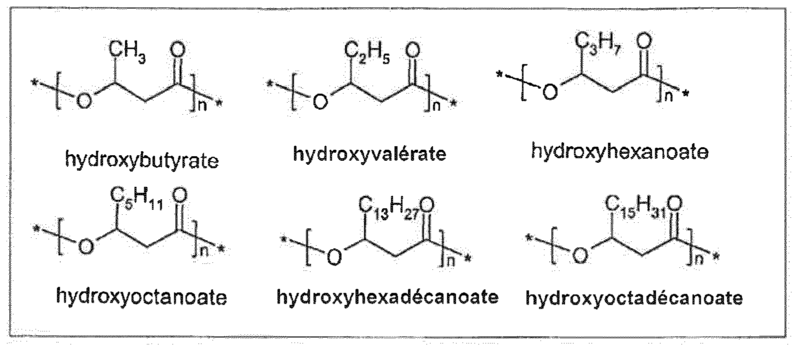

5. Procédé cosmétique selon l'une des revendications précédentes, dans lequel, dans la substance colorante, le bio-polyester comprend, comme motif de formule (I), un motif répétitif de formule (III), seul ou en mélange : 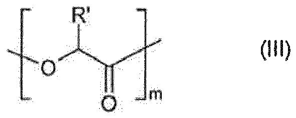 où
- R' est une chaîne C1-C18 alkyle linéaire ou ramifiée, ou un atome d'hydrogène ; et
- m est compris entre 450 et 1400.

6. Procédé cosmétique selon l'une des revendications précédentes, dans lequel, dans la substance colorante, les monomères de formule (I) représentent de 30% à 100% en poids, notamment de 40% à 95% en poids, voire de 55% à 90% en poids, et mieux encore de 70% à 80% en poids, du poids total du bio-polyester, préférablement 100% en poids.

7. Procédé cosmétique selon l'une des revendications précédentes, dans lequel la substance colorante est sous forme de poudre (solide), ayant une taille allant d'environ 500 nm à 50 microns, et mieux encore comprise entre 800 nm et 10 microns.

8. Procédé cosmétique selon l'une des revendications précédentes, dans lequel la substance colorante comprend de 40% à 95% en poids de bio-polyesters et de 5% à 60% en poids de composés colorants.

9. Procédé cosmétique selon l'une des revendications précédentes, dans lequel la substance colorante est présente dans la composition selon une proportion allant de 0,1% à 30% en poids, notamment de 0,5% à 25% en poids, voire de 1% à 20%, préférablement de 3% à 15% en poids, et mieux encore de 5% à 10% en poids, par rapport au poids total de la composition cosmétique.

10. Procédé cosmétique selon l'une des revendications précédentes, dans lequel le milieu cosmétiquement acceptable comprend au moins un ingrédient choisi parmi les solvants et/ou les huiles de silicone et/ou fluorés, volatils ou non volatils, carbonés, hydrocarbonés, d'origine minérale, animale, végétale ou synthétique ; les cires d'origine végétale, animale, minérale ou synthétique, ou les cires de silicone ; les pigments, les charges, les nacres et les paillettes brillantes ; les colorants liposolubles ou hydrosolubles ; les épaississants, les agents gélifiants, les antioxydants, les parfums, les huiles essentielles, les agents conservateurs, les agents actifs cosmétiques, les hydratants, les vitamines, les céramides, les écrans solaires, les agents d'enduction, les agents mouillants, les dispersants, les agents antimousse, les neutralisants, les stabilisants, les polymères et notamment les polymères filmogènes liposolubles ; les agents tensioactifs, les émulsifiants ; les charges, et des mélanges de ceux-ci.

11. Procédé cosmétique selon l'une des revendications précédentes, dans lequel la composition se trouve sous la forme d'un produit de soin ou de maquillage de la peau du corps ou du visage, des lèvres, des cils, des sourcils, des cheveux ou des ongles, d'un produit solaire ou autobronzant, ou d'un produit de soin capillaire, notamment pour la coloration, le conditionnement, le nettoyage, le coiffage et/ou le soin des cheveux.

12. Procédé cosmétique selon l'une des revendications précédentes, dans lequel la composition se trouve sous la forme d'un rouge à lèvres, d'une poudre pour le visage, d'un fard à paupières ou d'un fond de teint ; ou d'une composition solaire.

13. Procédé cosmétique selon l'une des revendications précédentes, pour le soin ou le maquillage des matières kératiniques, notamment de la peau du corps ou du visage, des lèvres, des ongles, des cheveux, des sourcils et/ou des cils.
